# EUROPÄISCHE PATENTSCHRIFT

(11) **EP 2 192 895 B1**
(45) Veröffentlichungstag und Bekanntmachung des Hinweises auf die Patenterteilung: **12.07.2017**
(21) Anmeldenummer: 08785584.7
(22) Anmeldetag: 16.08.2008
(51) Int. Cl.: A61K 9/00, A61K 9/70

(54) **TRANSDERMALES THERAPEUTISCHES SYSTEM ENTHALTEND LÄNGLICHE HOHLKÖRPER**
TRANSDERMAL THERAPEUTIC SYSTEM CONTAINING ELONGATE HOLLOW BODIES
SYSTÈME THÉRAPEUTIQUE TRANSDERMIQUE CONTENANT DES CORPS CREUX ALLONGÉS

(30) Priorität: 29.08.2007 DE 102007041557
(43) Veröffentlichungstag der Anmeldung: 09.06.2010
(73) Patentinhaber: LTS LOHMANN Therapie-Systeme AG, 56626 Andernach (DE)
(72) Erfinder: MAIER, Stephan, 51371 Leverkusen (DE)
(74) Vertreter: Siemund, Volker
(86) Internationale Anmeldenummer: PCT/EP2008/006742
(87) Internationale Veröffentlichungsnummer: WO 2009/030351

(56) Entgegenhaltungen:
- EP-A- 0 413 034
- WO-A-2005/016320
- DE-A1- 19 958 554

## Beschreibung

Die vorliegende Erfindung betrifft ein Transdermales therapeutisches System (TTS) zur Applikation eines Wirkstoffs oder mehrerer Wirkstoffe bzw. eines seiner therapeutisch wirksamen Salze an die Haut, wobei in dem TTS längliche Hohlkörper eingearbeitet sind, die ein Füllmedium oder mehrere Füllmedien aufweisen. Sie betrifft ferner die Herstellung und Verwendung derartiger Systeme.

Gefüllte Hohlfasern werden als Notschmiermittel in Schneid- oder Schleifwerkzeugen eingesetzt oder durch Freisetzung von Flüssigklebern zur Beseitigung von Haarrissen in Kunststoffen verwendet. Es ist inzwischen technisch möglich, die physikalischen Eigenschaften von länglichen Hohlkörpern dem spezifischen Einsatzzweck anzupassen. Dabei reicht das Spektrum z.B. von glasartig spröde bis gummielastisch flexibel, von gasdicht bis hochpermeabel. Der Außendurchmesser länglicher Hohlkörper kann dabei von unterhalb 10 nm (Nanoröhrchen) bis zu mehreren 100 µm (Hohlfasern) betragen.
Im Stand der Technik kommen bereits hohle Fasern in TTS zur Anwendung. So offenbart EP 0 227 836 A1 eine pharmazeutische Zubereitung, umfassend eine haftklebende Schicht mit Hohlfasern, die radial angebrachte Poren aufweisen. Dabei können die Wirkstoffe entweder in den Hohlfasern selbst eingebettet sein, oder aber die Hohlfasern sind leer und der Wirkstoff passiert über Diffusion eine Schicht aus Hohlfasern, um zur Hautoberfläche zu gelangen. In dieser Anwendung werden Hohlfasern in der Funktion einer Steuermembran verwendet, bzw. bewirken eine langsame kontrollierte Abgabe des Wirkstoffes.
JP 2004-168734 offenbart ein Nikotin-Pflaster zur Abgabe von Nikotin, wobei Hohlfasern mit offenen Poren, gefüllt mit einer Mischung aus Nikotin und einer niedermolekularen Substanz, verwendet werden. Die Wirkstoffabgabe aus dem TTS wird dadurch verzögert.
In EP 0 413 034 A1 wird ein gewebeartiger Verbund aus Hohlfasern mit offenen Poren als nicht okklusive Rückschicht eines TTS eingesetzt, um die Anreicherung von Feuchtigkeit im TTS gering zu halten, der durch den transepidermalen Wasserverlust entsteht und somit die Stabilität des Systems während der Tragedauer zu verbessern.

WO 2005/016320 A offenbart ein dermales oder transdermales therapeutisches System enthaltend eine Abdeckfolie mit Barriere Wirkung gegen Gase, Aromen und leicht flüchtige Stoffe, das dadurch gekennzeichnet ist, dass die Abdeckfolie aus mindestens einer Trägerschicht und einer Schicht aus einem anorganischorganischen Hybridpolymeren (Ormocer) besteht.

DE19958554 A1 offenbart die Verwendung von geeigneten physiologisch akzeptablen Lösemitteln, die die Beladung von Silikonklebern mit gelösten Wirkstoffen mittlerer Polarität verbessern und damit den Einsatzbereich von Silikonklebern und Mikroreservoir-Systemen erweitern.

Aufgabe der vorliegenden Erfindung war es, ein transdermales therapeutisches Systems (TTS) bereitzustellen, dass durch seinen einfachen Aufbau zum einen sicherstellt, dass das TTS sicher gelagert werden kann und eine Inaktivierung oder Aktivierung des Systems nicht bereits während der Lagerung geschieht. Zum anderen sollte die Erfindung ermöglichen, das System - je nach Ausführungsform - zu einem beliebigen Zeitpunkt (vor, nach oder während der Anwendung) bewusst (aktiv) oder automatisch (passiv) zu Aktivieren oder zu Deaktivieren.

Diese Aufgabe wird durch ein TTS gemäß Anspruch 1 in hervorragender Weise gelöst. Durch Zuführung von Strahlungsenergie oder anderer Energie wird eine Änderung der Struktur der Hohlkörper erzeugt, durch die das Füllmedium oder die Füllmedien aus den Hohlkörpern freigesetzt werden. Die Energie kann als mechanische, thermische, elektrische, magnetische, elektro-magnetische oder chemische Energie zugeführt werden. Dies ist von der jeweiligen Eigenschaften der Hohlkörper abhängig. Bevorzugt wird die Energie in Form von Druck, Wärme, Strahlung, Strom oder Schall zugeführt.

Zum Beispiel werden spröde Hohlkörper durch Biegen, Dehnen, Strecken, Verdrehen oder Knicken einer mechanischen Belastung ausgesetzt, durch die sie irreversible deformiert werden. Durch Brechen oder Bersten der Struktur der Hohlkörper im TTS wird das Füllmedium oder mehrere Füllmedien aus den länglichen Hohlkörpern freigesetzt und kann - je nach vorgesehener Ausführungsform - in verschiedener Weise reagieren. Der Grad der mechanischen Beanspruchung und damit der Beginn des Brechens oder Bersten der Hohlröhrchen hängt von der jeweiligen Anwendung und von dem Material der Hohlröhrchen ab. So ist es zum Beispiel möglich, ein TTS vor der Anwendung aktiv zu Knicken, um es zu aktivieren. In einer anderen Ausführungsform wird bei einer Abnahme des TTS von der Haut zumindest automatisch eine solch starke Biegung erreicht, dass die entsprechend dieser Anwendung eingesetzten Hohlröhrchen brechen und das freigesetzte Füllmedium, zum Beispiel nicht verbrauchten Wirkstoff, inaktiviert. In Anlehnung an die DIN EN 28510 werden die Hohlfasern gebrochen, wenn in einem Winkel von 180°, bevorzugt >90° das TTS z.B. von der Haut oder einem Substrat abgezogen wird. Auch während der Anwendung auf der Haut kann durch Druck auf das TTS durch einen Finger oder einen anderen Gegenstand ein (drucksensitives) Brechen erreicht werden und damit die Aktivierung erfolgen.

Bei flexiblen Hohlröhrchen funktioniert das oben beschriebene Prinzip nicht. Aber durch andere Energieformen, wie zum Beispiel Schall, Wärme, Strahlung o.ä., können als wirksame Mittel für ein Brechen, Bersten, Auflösen oder Perforieren der Hohlkörper genutzt werden. In einer bevorzugten Ausführungsform ist in den Hohlkörpern ein zusätzliches Mittel eingebracht, das sich durch Wärmeeintrag in das TTS ausdehnt und somit die Hohlkörper bersten. In einer weiteren bevorzugten Ausführungsform lösen sich die Hohlkörper aufgrund ihrer Materialeigenschaften bei Temperaturen oberhalb der Körpertemperatur auf oder werden perforiert. Eine Kombination aus spröden und flexiblen Hohlröhrchen mit verschiedenen Materialeigenschaften ist ebenfalls bevorzugt. Auf diese Weise können die sich in den Hohlkörpern befindlichen Wirkstoffe zum Beispiel zu definierten Zeitpunkten aktiviert werden.

In einer bevorzugten Ausführungsform umfasst das Füllmedium mindestens ein Wirkstoff und die Freisetzung des Wirkstoffs oder der Wirkstoffe erfolgt durch Brechen, Auflösen oder Perforieren der Hohlkörper. Durch die Separierung von Wirkstoffen und / oder Hilfsstoffen in der Matrix durch die Hohlröhrchen ergeben sich viele Vorteile, die im Folgenden anhand von Anwendungsbeispielen näher erläutert sind, ohne damit die Erfindung einzuschränken. Diese Beispiele dienen lediglich der Erläuterung der Erfindung.

Therapeutische Systeme (TTS) sind dem Fachmann grundsätzlich bekannt. Üblicherweise umfasst ein TTS eine wirkstoffundurchlässige Rückschicht, eine oder mehrere wirkstoffhaltige Schichten, optional eine die Wirkstofffreisetzung steuernde Membran und eine abziehbare Schutzschicht. Liegt eine Matrixschicht als wirkstoffhaltige Schicht vor, so kann sie selbstklebend ausgerüstet oder zusätzlich mit einer hautseitigen Haftkleberschicht versehen sein. Im Falle eines Wirkstoffreservoirs kann das Füllmedium als Lösung, Suspension, Gel oder Dispersion in einer festen Polymermatrix vorliegen. Eine die Wirkstofffreisetzung steuernde Membran ist dann zwischen der Haut und dem Wirkstoffreservoir eingefügt, wobei die Membran wiederum mit einer hautseitigen Haftkleberschicht zur Fixierung auf der Haut versehen ist. Geeignet sind ebenfalls Mikroreservoirsysteme, die den Wirkstoff in mikroverkapselter Form enthält. Die wirkstoffhaltige Schicht im Sinne der Erfindung umfasst zumindest einen oder mehrere Wirkstoffe und die länglichen Hohlkörper mit Füllmedium, wobei sich der oder die Wirkstoffe sowohl innerhalb wie auch außerhalb der Hohlkörper befinden können. Das Füllmedium liegt in gasförmiger, flüssiger, halbfester oder fester Form vor. Das System kann als mehrschichtiges System vorliegen, wobei in den verschiedenen Schichten, gleiche oder verschiedene Wirkstoffe vorliegen können, aber auch in unterschiedlichen Konzentrationen. Bevorzugt sind erfindungsgemäße Wirkstoffpflaster, wobei die resultierenden TTS nach Applikation auf der Haut transparent oder zumindest transluzent (d. h. durchscheinend) sind.

Die Rückschicht muss für den im TTS enthaltenen Wirkstoff undurchlässig sein, um ein unerwünschtes Austreten des Wirkstoffs zu verhindern. Als Rückschicht eigenen sich insbesondere Polyester, wie z. B. bevorzugt Polyethylenterephthalat (PET) und Polybutylenterephthalat, welche sich durch besondere Festigkeit auszeichnen. Darüber hinaus sind nahezu beliebige andere verträgliche Kunststoffe, wie z.B. Polyvinylchlorid, Ethylenvinylacetat, Vinylacetat, Polyethylen, Polypropylen, Cellulosederivate oder Kombinationen verschiedener Folien geeignet. Häufig werden Verbundlaminate aus Aluminium und Kunststoffmaterialien wie Polyethylenterephthalat eingesetzt. Der Vorteil dieser Verbundlaminate liegt darin, dass Aluminiumfolien kostengünstig herzustellen und nahezu gegenüber allen pharmazeutischen Wirkstoffen undurchlässig sind. Zudem sind Aluminiumfolien lichtundurchlässig, was gerade bei lichtempfindlichen Wirkstoffen den Vorteil eines zuverlässigen Schutzes vor Licht bietet.

Als Steuermembran können mikroporöse, mit definierter Porengröße hergestellte Polymerfolien aus Polyethylen, Polypropylen, Polyurethan, Copolymeren aus Ethylen und Vinylacetat (EVA) und Silikonen verwendet werden. Geeignet sind diese Polymerfolien, sofern sie beständig gegenüber den in der Wirkstoffzubereitung enthaltenen Substanzen sind.

Als Haftkleber eignen sich hochviskose Stoffe, die nach kurzem leichtem Druck auf der Haut kleben, so genannte druckempfindliche Haftkleber (PSA). Sie besitzen hohe Kohäsions- und Adhäsionskräfte. Zum Einsatz kommen Haftkleber auf Basis von Poly(meth)acrylaten, auf Basis von Polyisobutylenen und auf Basis von Silikonen. Die Haftklebeschicht kann wirkstoffhaltig oder wirkstofffrei sein. Im Sinne der Erfindung kann die Haftklebeschicht die wirkstoffhaltige Schicht sein und somit auch die länglichen Hohlkörper umfassen. Der Wirkstoff kann oder die Wirkstoffe können somit lediglich in der Schicht oder nur in den länglichen Hohlkörper vorkommen. Die Wirkstoffe können aber auch sowohl in der Schicht und in den länglichen Hohlkörpern sein.
Die Haftkleberschicht und / oder optional vorhandene(n) Reservoir- bzw. Matrixschicht(en) des erfindungsgemäßen TTS kann/können ein Material umfassen, das aus der Gruppe ausgewählt ist, die aus haftklebenden Polymeren auf Basis von Acrylsäure und/oder Methacrylsäure sowie deren Estern, Polyacrylaten, Isobutylen, Polyvinylacetat, Ethylen-Vinylacetat, natürlichen und/oder synthetischen Kautschuken, beispielsweise Acrylnitril-Butadien-Kautschuk, Butylkautschuk oder Neoprenkautschuk, Styrol-Dien-Copolymeren wie Styrol-Butadien-Blockcopolymeren und Heißschmelzklebern besteht, oder das auf Basis von haftklebenden Silikonpolymeren oder Polysiloxanen hergestellt ist.
Polyacrylate werden generell durch Polymerisation verschiedener Monomere (mindestens ein Monomer aus der Gruppe umfassend Acrylsäure, Methacrylsäure, Acrylsäureestern und Methacrylsäureestern) und insbesondere aus deren Mischungen hergestellt. Als Lösungsmittel bei der Polymerisation zur Herstellung eines geeigneten Polyacrylats werden vorzugsweise organische Lösungsmittel, in manchen Fällen auch Wasser verwendet. In Abhängigkeit von der Struktur der bei der Polymerisation eingesetzten Monomere erhält man Polyacrylate, die funktionelle Gruppen enthalten können. Weit verbreitet sind Polyacrylate mit OH-Gruppen (Hydroxylgruppen) und solche mit COOH-Gruppen (Carboxylgruppen) als funktionellen Gruppen.
Als geeignete Silikonpolymere kommen Silikone, Polysiloxane oder Silikone, die selbst haftklebend sein können, zur Anwendung. Als Silikonhaftkleber werden Haftkleber z.B. basierend auf einer Polydimethylsiloxanstruktur oder Polydimethyldiphenylsiloxanstruktur verstanden. Insbesondere kommerziell erhältliche SilikonHaftkleber, wie z.B. BIO-PSA von Dow Corning Corporation sind geeignet.

Die Schutzschicht dient zum Schutz der hautseitigen Haftklebeschicht des Pflasters während der Lagerung und wird kurz vor der Applikation entfernt.
Es handelt sich im Allgemeinen um abhäsiv ausgerüstete Folien, wie zum Beispiel einseitig silikonisierte Polyesterfolien aus Polyetyhlenterephthalat (PET).

Als Wirkstoffe kommen alle die Wirkstoffe in Betracht, die für eine transdermale oder topische Applikation geeignet sind. Darunter werden im Sinne der Erfindung chem. Substanzen, Arzneistoffe, Duftstoffe, Pheromone verstanden. Der Wirkstoff oder die Wirkstoffe können auch in Form seiner pharmakologisch wirksamen Salze oder als Prodrug vorliegen. Unter einem Prodrug wird ein Stoff verstanden, der ohne Verstoffwechslung pharmakologisch nicht oder wenig wirksam ist und erst durch die Verstoffwechslung im Körper in einen aktiven Wirkstoff überführt wird.

In einer besonderen Ausführungsform befinden sich die empfindlichen und/oder leichtflüchtigen Wirkstoffe in den länglichen Hohlkörpern. Damit ist die Gefahr des Wirkstoffverlustes vor Anwendung durch Diffusionsprozesse vermieden, zumindest aber erheblich eingeschränkt. Wie bereits beschrieben, können die Wirkstoffe in verschiedenen Schichten vorkommen, wobei mindestens in einer der Schichten die länglichen Hohlkörper enthalten sind. Bei dem Wirkstoff handelt es sich bevorzugt um einen Wirkstoff aus der Gruppe der Schmerzmittel wie z. B. Narkotika. Bevorzugt zu nennen sind Morphinderivate, Heroin und Buprenorphin, oder Fentanyl, Sufentanyl und Alfentanyl oder ihre Derivate. Empfindliche und/oder leichtflüchtige Wirkstoffe werden ebenfalls bevorzugt eingesetzt, wie zum Beispiel Nikotin, Nitroglycerin, Salicylsäure, Scopolamin, Benzatropin, Bupropion, Cyclopentamin Dapsaicin, Fenfluramin, Fentanyl, Cyclopentamin, Ephedrin, Selegilin, Sufentanil, Oxybutynin, Rasagilin, Mecamylamin, Memantin, Methylsalicylat, Venlafaxin, und andere.

Die wirkstoffhaltige Schicht kann neben dem Wirkstoff bzw. den Wirkstoffen und den länglichen Hohlkörpern zusätzlich noch weitere Hilfsstoffe, wie Weichmacher, Klebrigmacher, Lösungsvermittler, Stabilisatoren, Füllstoffe, Trägerstoffe, Permeations-Beschleuniger, wie zum Beispiel Fettalkohole, Polyolfettsäureester, Polyalkohole, Azone, Alkylmethylsulfoxide, Pyrrolidon, nichtionische Tenside, anionische Tenside, kationische Tenside, Terpene enthalten, die dem Fachmann grundsätzlich bekannt sind. Weitere bevorzugte Hilfsstoffe der vorliegenden Erfindung sind z. B. Silikonöl, Glycerinester von hydrierten Harzsäuren, Hydroabietin-Alkohol-Harz-Ester, Hydroabietin-Säure-Harz-Ester, hydrogenierte Methylester von Terpentinharzen, Ester von partiell hydrierten Terpentinharzen, Ester von Terpentinharzen, Colophoniumharz, Phenolharz, Alkylphenolharz, Petroleumharz, Xylolharz. Alternativ können zusätzlich noch Antioxidantien, wie Tocopherole, Butylhydroxyanisol (BHA), Gallussäureester, Butylhydroxytoluol (BHT), Ascorbylpalmitat, -stearat, zur Stabilisierung bzw. Minimierung des Wirkstoffabbaus während der Lagerung eingesetzt werden. Die Wirkstoffzubereitung kann zusätzlich viskositätserhöhende Hilfsstoffe enthalten, die keine die Freisetzung steuernde Funktion haben. Vorzugsweise ist der viskositätserhöhende Hilfsstoff aus der Gruppe ausgewählt, die aus feindispersem Siliziumdioxid, beispielsweise Aerosil R 974^{®}, Metalloxide, Titanoxid, Zinkoxid, Silikat, Aluminium-, Magnesiumsilikat, Talkum, Stearat, Zinkstearat, Polyethylen, polystyrolunlösliches Polyvinylpyrrolidon, Polyacrylsäuren, z. B. Carbopol 934P^{®}, Mineralölen, Wollwachsen und hochmolekularen Polyethylenglykolen besteht. Ein Beispiel für ein bevorzugtes Polyethylenglykol ist Carbowax 1000^{®}. Durch Zugabe geeigneter Lösungsmittel, sowie gegebenenfalls weiterer Hilfsstoffe, kann die Viskosität der Wirkstoffzubereitung eingestellt werden. Als Lösungsmittel sind prinzipiell alle gängigen organischen Lösungsmittel geeignet, wie zum Beispiel Ethanol, Isopropylalkohol, Hexan, Heptan, Ethylacetat, Petrolether, Benzin, Ketone, Aceton, Glycerol, DEET (N,N-Diethyl-3-methylbenzamid), THF sowie viele Öle, beispielsweise Silikonöl, Paraffin, Triglyceride, Neutralöl oder pflanzliche Öle. Die transdermalen therapeutischen Systeme können zur verbesserten Lösung des Wirkstoffs im Polymer ein oder mehrere Lösungsmittel enthalten. Hierfür kommen lösliches Polyvinylpyrrolidon, Kollidon-Vinylacetat, Propylenglykol, Ethyloleat, 1,2-Propandiol, 1,3-Butandiol, Transcutol, Propylenglykolmonocaprylat, Solketal, Ölsäure, 1-Methyl-pyrrolidon, Glycerol, Lauryllactat, Triacetin, Glycerolmonooleat, Sorbitanmonooleat und Sorbitantrioleat in Frage. Besonders bevorzugt sind Propylenglykol, Butandiol und Lauryllactat.

Unter einem länglichen Hohlkörper im Sinne dieser Anmeldung wird ein Gegenstand verstanden, dessen Längenausdehnung den seines Durchmessers übersteigt. Typischerweise besitzen sie eine Länge von einigen Mikrometern. Es stehen aber schon Hohlkörper bis zu 20 Zentimetern zu Verfügung. Bei der Verwendung von Hohlkörpern in Form von Fließkissen, Pads oder Kompressen können die Produkte auch bis zu mehreren Metern lang sein. Der Außendurchmesser der Hohlkörper beträgt 1 nm bis zu 1000 µm, bevorzugt 10 nm bis 500 µm, besonders bevorzugt 10 nm bis 100 µm, ganz besonders bevorzugt 10 nm bis 50 µm. Es werden zum Beispiel Hohlfasern, Nanofasern, Nanoröhrchen, Buckytubes, Nanotubes, nanoskalige Kohlenstoffröhren, Carbon Nanotubes (CNTs) eingesetzt.
Die Hohlkörper können ein- oder mehrwandig sein, wobei die Wand einen geschlossenen Ring oder eine spiralige Struktur ausbildet, wobei der Querschnitt über die Länge der Hohlkörper weitgehend gleich bleibt und vorzugsweise kreisförmig ist. Die Wandstärke ist von der Art der Herstellung abhängig und liegt bevorzugt im Bereich von 5 bis 10 % des Außendurchmessers. Bevorzugt sind sie nicht mit Poren versehen und somit als undurchlässig für das Füllmedium zu verstehen. In den Hohlkörpern ist ein Füllmedium oder mehrere Füllmedien vorgesehen, wie zum Beispiel ein Gas oder eine Mischung verschiedener Gase, eine Flüssigkeit oder mehrere Flüssigkeiten (Lösungen, Suspensionen, Emulsionen), ein Feststoff oder mehrere Feststoffe oder halbfesten Zubereitung (Pasten, Gele, Salben, Cremes, etc.). Da in der bevorzugten Ausführung der Durchmesser verschwindend klein gegenüber der Längenausdehnung ist, ist hinsichtlich der Dichtigkeit unerheblich, ob die Enden der Hohlkörper offen sind.
Die bevorzugten Hohlkörper sind nur in einem - je nach Ausführungsform - vorbestimmten Maß plastisch verformbar, bis Risse entstehen und die Hohlkörper schließlich brechen. Sie besitzen daher eine Sprödigkeit. Sie brechen bei einem vorbestimmten, über die Zusammensetzung des Wandmaterials der Hohlkörper einstellbaren Biegeradius bzw. Winkel, z.B. beim Abziehen des TTS von der Haut oder durch Fingerdruck bzw. einen anderen Gegenstand auf das auf der Haut befindlichen TTS. Damit wird ein (drucksensitives) Brechen erreicht und die Aktivierung oder Deaktivierung des Systems gestartet. Aber auch andere Möglichkeiten, wie zum Beispiel Schall, Wärme, Strahlung o.ä., können als wirksame Mittel für eine Zerstörung der Hohlkörper genutzt werden. In einer solchen Ausführungsform sind die mechanischen Eigenschaften der Hohlfasern im gesamten Spektrum von glasartig spröde bis gummiartig elastisch denkbar.
Der Wahl der Materialien sind keine Grenzen gesetzt. Üblicherweise bestehen sie aus Kohlenstoff, Metallen, Metalloxiden oder Polymerverbindungen oder deren Kombinationen. Besonders bevorzugt verwendet wird eine neue Werkstoffgruppe, die ORMOCERe (organically modified ceramics), bei der glasartige mit polymeren Komponenten verbunden sind. Der Verbundwerkstoff besteht aus einem anorganischen Silizium-Sauerstoff-Netz in das spezielle organische vernetzbare Molekülgruppen eingebaut sind. Die anorganischen, glasartigen Strukturen ermöglichen das Spinnen des Materials. Die organischen Segmente sorgen dafür, daß die Fasern sich in ihren Eigenschaften problemlos modifizieren lassen. So reicht das Eigenschaftsspektrum der ORMOCER-Hohlfasern von glasartig-spröde bis gummi-elastisch flexibel, von gasdicht bis hochdurchlässig. In einer besonderen Ausführungsform umfaßt die wirkstoffhaltige Schicht neben den spröden Hohlkörpern zusätzlich noch flexible Hohlkörper. Diese können durch Anwendung von Schall, Wärme oder Strahlung als auflösendes Prinzip aktiviert werden und ihren Inhalt freigeben.
Die Herstellung von Hohlfasern erfolgt nach den dem Fachmann bekannten Techniken z.B. durch Spinnverfahren, wobei mit Druckluft ein Harz durch eine Hohlspinndüse gedrückt wird. Anschließend wird die Faser durch UV-Strahlung ausgehärtet. Weitere Verfahren sind z.B. Elektrospinnen, bei dem an eine Düse, durch die eine Kunststofflösung oder Schmelze gepumpt wird, eine hohe Spannung angelegt wird, das TUFT-Verfahren (Tubes By Fiber Templates), dass auf der Beschichtung von Templatfasern beruht und das Templat nach Hohlkörperbildung selektiv entfernt wird, oder das WASTE-Verfahren (Wetting Assisted Templating) bei dem die Wände der Poren eines porösen Templats mit einer Polymerlösung oderschmelze benetzt werden. Anschließend können die so erhaltenen Hohlkörper durch selektive Einführung funktioneller Gruppen an der Innen- oder Außenwand der Hohlkörper weiter funktionalisiert werden.
- Die so hergestellten länglichen Hohlkörper lassen sich entweder als reines Schüttgut in das TTS einarbeiten, oder aber als ganze Bündel zu Fäden oder Matten verarbeiten und als flächige Schichten, als Filme, als Laminate oder Gewebe, gewebte flächenartige Pads verwenden, z.B. als flächenförmiges, z.B. gewebtes Material aus gefüllten länglichen Hohlkörpern in Bahn- bzw. Rollenform, aus dem die zu verwendende Kontur über einen kombinierten Siegel und / oder Stanzprozess gewonnen wird, ohne dass das Füllmedium aus den länglichen Hohlkörpern austritt.
- als folienförmiges Material, bei dem die gefüllten länglichen Hohlkörper in ein Trägermedium, z.B. aus Kunststoff wie z.B. Polyurethan eingebettet sind.
- als flächige Schichten aus gefüllten länglichen Hohlkörpern (Pads) mit definierten Abmessungen zwischen 0,1 und 200 cm².

Zum Einarbeiten der Hohlkörper in Form der genannten flächigen Schichten stehen die dem Fachmann hinreichend bekannten vielfältigen Techniken der TTS Herstellung zur Verfügung. Die gefüllten länglichen Hohlkörper werden in das TTS in Form eines Schüttgutes, durch
- direktes Einbringen in die Beschichtungsmasse vor dem Beschichtungsprozess
- direktes Einbringen in das Wirkstoffreservoir vor dem Beschichtungsprozess
- Aufbringen des Schüttgutes auf die ungetrocknete Klebermatrix
- Aufbringen des Schüttgutes auf die getrocknete Klebermatrix
eingebettet. Bevor das Laminat mit der Rückschicht abgedeckt wird, kann eine Abdeckung der Hohlkörperschicht durch eine oder mehrere weitere Schichten erfolgen.

Im einfachsten Fall ist das Füllmedium ein Wirkstoff, der in den länglichen Hohlkörpern untergebracht ist. Durch Knicken oder Verdrehen des TTS werden die Hohlkörper gebrochen und der Wirkstoff aus den Hohlkörpern freigesetzt. Diese Konstruktion gewährleistet eine sichere Lagerung. Die Freisetzung kann aber auch durch Auflösen oder Perforieren der Hohlkörper erfolgen, hängt aber von den Eigenschaften der Hohlkörper ab. Durch die bereitgestellte Konstruktion des TTS wird tatsächlich erst vor der Anwendung das System gezielt aktiviert. Besonders vorteilhaft ist diese Ausführungsform, wenn es sich um empfindliche oder leichtflüchtige Wirkstoffe, insbesondere Nikotin handelt, oder eine Inkompatibilität zu einem anderen Inhaltsstoff des Systems besteht. Beispiele solcher Wirkstoffe sind insbesondere Nikotin, Nitroglycerin, Salicylsäure, Scopolamin, Benzatropin, Fenfluramin, Cyclopentamin, Ephedrin, Selegilin, Rasagilin, Mecamylamin, Memantin und andere.

Üblicherweise wird missbräuchlich versucht, durch Extraktion von getragenen TTS an den schmerzstillenden Wirkstoff heranzukommen. In einer besonderen Ausführungsform wird durch den Erfindungsgegenstand das Missbrauchspotential opioidhaltiger Schmerzpflaster deutlich gesenkt. In dieser Ausführungsform befindet sich der Wirkstoff in der Matrix und in den Hohlkörpern als Füllmedium ein Stoff, der den Wirkstoff bei Kontakt zerstört oder inaktiviert. Wird nun das TTS von der Haut entfernt, so kommt es durch das Abziehen und der damit verbundenen starken mechanischen Beanspruchung (Biegen) des TTS zum Brechen der Hohlkörper. Der Stoff wird freigesetzt und kommt mit dem Wirkstoff in Kontakt. Dieser wird durch zum Beispiel Oxidationsprozesse irreversibel zerstört oder durch Freisetzung eines Antagonisten inaktiviert. Als Oxidationsmittel eignen sich insbesondere anorganische Reagenzien, wie Permanganate, z.B. Kaliumpermanganat, Mangandioxid, Bleidioxid, Bleitetraacetat, Cer(IV)-Salze, Chromate, Chromsäure, Osmiumtetroxid, Salpetersäure, Nitrite, wie Kaliumnitrit, Selendioxid, Wasserstoffperoxid und andere Peroxo-Verbindungen, Brom, Chlor, Hypohalogenide, oder Schwefel; bevorzugt Kaliumpermanganat, Wasserstoffperoxid und Kaliumnitrit; organische Oxidantien, wie Dimethylsulfoxid, N-Bromsuccinimid, Chinone, hypervalente lodverbindungen, Persäuren und Perester, aber auch Enzyme. Bei gegebenem Wirkstoff wird das Oxidationsmittel bevorzugt aufgrund seiner chemischen Reaktionsfähigkeit mit dem Wirkstoff ausgewählt. Eine missbräuchliche Isolierung des Wirkstoffes ist somit wirksam unterbunden. Bei dem Wirkstoff handelt es sich bevorzugt um einen Wirkstoff aus der Gruppe der Schmerzmittel wie z. B. Narkotika. Bevorzugt sind zu nennen Morphinderivate, Heroin und Buprenorphin, oder Fentanyl und seine Derivate Sufentanyl und Alfentanyl. Grundsätzlich sind auch Wirkstoff-Kombinationen verwendbar, für die die Applikation über ein TTS die geeignete Darreichungsform ist.

In einer weiteren bevorzugten Ausführungsform liegen in der wirkstoffhaltigen Matrixschicht mindestens zwei Wirkstoffe vor, wobei sich zumindest einer der Wirkstoffe in den länglichen Hohlkörpern befindet. Diese Aufteilung ist dann von Vorteil, wenn zumindest zwei nicht miteinander kompatible Wirkstoffe vorliegen und erst kurz vor der Anwendung zusammen kommen sollen. Ein besonderer Vorteil, der sich aus dieser Konstruktion ergibt, ist, dass das Brechen, Auflösen oder Perforieren der Hohlkörper bevorzugt erst nach einem vorgegebenen Zeitintervall, z.B. nach 6 bis 36 bis hin zu 168 Stunden, erfolgt und dann der Wirkstoff zeitversetzt zum ersten freigesetzt wird. Es ist somit ein Zwei-Phasen TTS zur zweimaligen Applikation bei gleichzeitiger Verwendung desselben TTS zur wiederholten Anwendung. Nach der ersten Trageperiode wird die zweite Phase des TTS durch Brechen der Hohlkörper (durch den Anwender) auf der Haut oder nach Entfernen des TTS und erneutes Aufkleben des TTS gestartet.

In einer weiteren bevorzugten Ausführungsform liegt in der wirkstoffhaltigen Schicht zumindest ein Wirkstoff in Form eines seiner pharmakologisch wirksamen Salze vor. In den länglichen Hohlkörpern befindet sich als Füllmedium ein basischer Hilfsstoff, der den Wirkstoff aus der Salzform freisetzt und den somit in die pharmakologisch wirksame oder wirksamere Form überführt. Diese bevorzugte Ausführungsform der Erfindung ist somit die In-situ Erzeugung einer Wirkstoff-Base durch Freisetzung durch den basischen Hilfsstoff vor der Anwendung des TTS. Auch die umgekehrte Anordnung, (Wirkstoff in der Salzform als Füllmedium in den länglichen Hohlkörpern und basischer Hilfsstoff in der Schicht) ist eine bevorzugte Ausführungsform dieser Erfindung.

Eine weitere besonders bevorzugte Ausführungsform sieht vor, dass die Wände der länglichen Hohlkörper semipermeabel ausgestaltet sind. Wasser in Form von Hautfeuchtigkeit kann somit während der Tragedauer des TTS in die Hohlkörper aufgenommen werden. Durch Wasserzutritt in den Hohlkörper mittels Diffusion von Lösemittel durch die semipermeable Membran werden diese so weit aufgeweitet, dass die Hohlkörper bersten und das Füllmedium freisetzen. Zusätzlich können noch semipermeabel nicht gefüllten Hohlkörper im TTS vorhanden sein, durch die vorteilshafterweise eine Reduktion der Klebkraft des TTS oder einen Abbau des Wirkstoffs während der Tragedauer durch den transepidermalen Wasserverlust der Haut verhindert oder zumindest verringert wird.

## Patentansprüche

1. Transdermales therapeutisches System umfassend eine wirkstoffundurchlässige Rückschicht, eine wirkstoffhaltige Schicht oder mehrere wirkstoffhaltige Schichten, optional eine ablösbare Schutzschicht, wobei mindestens eine der wirkstoffhaltigen Schichten längliche, mit einem Füllmedium oder mehreren Füllmedien versehene Hohlkörper enthält, **dadurch gekennzeichnet, dass** das Füllmedium oder die Füllmedien durch Änderung der Struktur der Hohlkörper aus diesen freisetzbar ist bzw. sind.

2. Transdermales therapeutisches System nach Anspruch 1, **dadurch gekennzeichnet, dass** die Änderung der Struktur der Hohlkörper durch Zuführung von mechanischer, thermischer, elektrischer, magnetischer, elektromagnetischer oder chemischer Energie bewirkt wird.

3. Transdermales therapeutisches System nach Anspruch 1 oder 2, **dadurch gekennzeichnet, dass** die Änderung der Struktur der Hohlkörper durch Druck, Wärme, Strahlung, Strom oder Schall hervorgerufen wird, bevorzugt durch Druck oder Wärme.

4. Transdermales System nach einem oder mehreren der vorangehenden Ansprüche, **dadurch gekennzeichnet, dass** das Füllmedium mindestens einen Wirkstoff umfasst und die Freisetzung des Wirkstoffs oder der Wirkstoffe durch Brechen, Auflösen oder Perforieren der Hohlkörper erfolgt.

5. Transdermales System nach einem oder mehreren der vorangehenden Ansprüche, **dadurch gekennzeichnet, dass** die wirkstoffhaltige Schicht mindestens zwei Wirkstoffe enthält, wobei sich zumindest einer der Wirkstoffe in den länglichen Hohlkörpern befindet und durch Brechen, Auflösen oder Perforieren der Hohlkörper freigesetzt wird, bevorzugt nach einem vorgegebenen Zeitintervall.

6. Transdermales therapeutisches System nach einem oder mehreren der vorangehenden Ansprüche, **dadurch gekennzeichnet, dass** das sich im Hohlkörper befindliche Füllmedium den Wirkstoff oder die Wirkstoffe inaktivierende Eigenschaft hat und bevorzugt ein Antagonist ist.

7. Transdermales therapeutisches System nach einem oder mehreren der vorangehenden Ansprüche, **dadurch gekennzeichnet, dass** das sich im Hohlkörper befindliche Füllmedium den Wirkstoff oder die Wirkstoffe zerstörende Eigenschaft hat und bevorzugt ein Oxidationsmittel ist.

8. Transdermales therapeutisches System nach den Ansprüchen 6 oder 7 **dadurch gekennzeichnet, dass** das Füllmedium erst durch die mechanische Beanspruchung des TTS beim Entfernen von der Haut aus den Hohlkörpern freigesetzt wird, bevorzugt durch Brechen der Hohlkörper.

9. Transdermales System nach einem oder mehreren der vorangehenden Ansprüche, **dadurch gekennzeichnet, dass** die wirkstoffhaltige Schicht mindestens einen Wirkstoff in Form eines seiner therapeutisch wirksamen Salze enthält, wobei sich der den Wirkstoff aus der Salzform freisetzende basische Hilfsstoff in den Hohlkörpern befindet.

10. Transdermales System nach einem oder mehreren der vorangehenden Ansprüche, **dadurch gekennzeichnet, dass** die wirkstoffhaltige Schicht ein den Wirkstoff aus der Salzform freisetzenden basischen Hilfsstoff enthält, wobei sich mindestens ein Wirkstoff in Form eines seiner therapeutisch wirksamen Salze in den Hohlkörpern befindet.

11. Transdermales System nach einem oder mehreren der vorangehenden Ansprüche, **dadurch gekennzeichnet, dass** die Wand der Hohlkörper semipermeable ausgestaltet ist und durch ein bevorzugt durch Wasserzutritt in die Hohlkörper hervorgerufenes Brechen dieser das Füllmedium freisetzt.

12. Transdermales therapeutisches System nach einem oder mehreren der vorangehenden Ansprüche, **dadurch gekennzeichnet, dass** der Außendurchmesser der Hohlkörper von 1 nm bis zu 1000 µm, beträgt, bevorzugt 10 nm bis 500 µm, besonders bevorzugt 10 nm bis 100 µm, ganz besonders bevorzugt 10 nm bis 50 µm.

13. Transdermales therapeutisches System nach einem oder mehreren der vorangehenden Ansprüche, **dadurch gekennzeichnet, dass** die Hohlkörper spröde und / oder flexibel ausgestaltet sind.

14. Transdermales therapeutisches System nach einem oder mehreren der vorangehenden Ansprüche **dadurch gekennzeichnet, dass** die Hohlkörper aus ORMOCER-Hohlfasern hergestellt sind.

15. Transdermales System nach einem oder mehreren der vorangehenden Ansprüche, **dadurch gekennzeichnet, dass** die wirkstoffhaltige Schicht zusätzliche eine hautseitige Haftkleberschicht aufweist.

## Claims

1. Transdermal therapeutic system comprising a backing that is impermeable to the active substance(s), a layer containing the active substance(s) or several layers containing the active substance(s), optionally a detachable protective layer, with at least one of the layers containing the active substance(s) having elongated hollow bodies provided with a filler or several fillers, **characterized in that** the filler or fillers can be released from the hollow bodies by changing the structure of the latter.

2. Transdermal therapeutic system according to Claim 1, **characterized in that** the change in structure of the hollow bodies is brought about by supplying mechanical, thermal, electrical, magnetic, electromagnetic or chemical energy.

3. Transdermal therapeutic system according to Claim 1 or 2, **characterized in that** the change in structure of the hollow bodies is caused by pressure, heat, radiation, electric current or sound, preferably by pressure or heat.

4. Transdermal system according to one or more of the preceding claims, **characterized in that** the filler comprises at least one active substance and the release of the active substance or active substances is effected by breaking, disintegrating or perforating the hollow bodies.

5. Transdermal system according to one or more of the preceding claims, **characterized in that** the layer containing the active substance(s) contains at least two active substances, where at least one of the active substances is located in the elongated hollow bodies and is released by breaking, disintegrating or perforating the hollow bodies, preferably after a given time interval.

6. Transdermal therapeutic system according to one or more of the preceding claims, **characterized in that** the filler contained in the hollow bodies has the property of inactivating the active substance or active substances and is preferably an antagonist.

7. Transdermal therapeutic system according to one or more of the preceding claims, **characterized in that** the filler contained in the hollow bodies has the property of destroying the active substance or active substances and is preferably an oxidizing agent.

8. Transdermal therapeutic system according to Claims 6 or 7, **characterized in that** the filler is only released from the hollow bodies by mechanical stressing of the TTS during removal from the skin, preferably as a result of breaking the hollow bodies.

9. Transdermal system according to one or more of the preceding claims, **characterized in that** the layer containing the active substance(s) contains at least one active substance in the form of one of its therapeutically effective salts, and the basic excipient that releases the active substance from the salt form is contained in the hollow bodies.

10. Transdermal system according to one or more of the preceding claims, **characterized in that** the layer containing the active substance(s) contains a basic excipient that releases the active substance from the salt form, with at least one active substance in the form of one of its therapeutically effective salts being located in the hollow bodies.

11. Transdermal system according to one or more of the preceding claims, **characterized in that** the wall of the hollow bodies is designed to be semipermeable and the filler is released preferably through breaking of the hollow bodies caused by supply of water to the latter.

12. Transdermal therapeutic system according to one or more of the preceding claims, **characterized in that** the outside diameter of the hollow bodies is from 1 nm to 1000 µm, preferably 10 nm to 500 µm, especially preferably 10 nm to 100 µm, and quite especially preferably 10 nm to 50 µm.

13. Transdermal therapeutic system according to one or more of the preceding claims, **characterized in that** the hollow bodies are designed to be brittle and/or flexible.

14. Transdermal therapeutic system according to one or more of the preceding claims, **characterized in that** the hollow bodies are made from ORMOCER hollow fibers.

15. Transdermal system according to one or more of the preceding claims, **characterized in that** the layer containing the active substance(s) additionally has a pressure-sensitive adhesive layer on the side next to the skin.

## Revendications

1. Système thérapeutique transdermique comprenant une couche arrière imperméable aux substance actives, une couche contenant des substances actives ou plusieurs couches contenant des substances actives, en option une couche de protection amovible, dans lequel au moins une des couches contenant des substances actives contient des corps allongés dotés d'un fluide de remplissage ou de plusieurs fluides de remplissage, **caractérisé en ce que** le fluide de remplissage ou les fluides de remplissage peut ou peuvent être libéré(s) hors des corps creux par un changement de la structure de ceux-ci.

2. Système thérapeutique transdermique selon la revendication 1, **caractérisé en ce que** le changement de la structure des corps creux est provoqué par l'application d'une énergie mécanique, thermique, électrique, magnétique, électromagnétique ou chimique.

3. Système thérapeutique transdermique selon la revendication 1 ou 2, **caractérisé en ce que** le changement de la structure des corps creux est provoqué par la pression, la chaleur, un rayonnement, un courant ou un son, de préférence par la pression ou la chaleur.

4. Système thérapeutique transdermique selon une ou plusieurs des revendications précédentes, **caractérisé en ce que** le fluide de remplissage comprend au moins une substance active et la libération de la substance active ou des substances actives se produit par rupture, dissolution ou perforation des corps creux.

5. Système thérapeutique transdermique selon une ou plusieurs des revendications précédentes, **caractérisé en ce que** la couche contenant des substances actives contient au moins deux substances actives, dans lequel au moins une des substances actives se trouve dans les corps creux allongés et est libérée par rupture, dissolution ou perforation des corps creux, de préférence après un intervalle de temps prédéterminé.

6. Système thérapeutique transdermique selon une ou plusieurs des revendications précédentes, **caractérisé en ce que** le fluide de remplissage se trouvant dans le corps creux a une propriété d'inactivation de la substance active ou des substances actives et est de préférence un antagoniste.

7. Système thérapeutique transdermique selon une ou plusieurs des revendications précédentes, **caractérisé en ce que** le fluide de remplissage se trouvant dans le corps creux a une propriété de destruction de la substance active ou des substances actives et est de préférence un agent oxydant.

8. Système thérapeutique transdermique selon une des revendications 6 ou 7, **caractérisé en ce que** le fluide de remplissage n'est libéré hors des corps creux que par la sollicitation mécanique du STT lors de l'enlèvement de la peau, de préférence par rupture des corps creux.

9. Système thérapeutique transdermique selon une ou plusieurs des revendications précédentes, **caractérisé en ce que** la couche contenant des substances actives contient au moins une substance active sous la forme d'un de ses sels thérapeutiquement actifs, dans lequel l'adjuvant basique libérant la substance active de la forme de sel se trouve dans les corps creux.

10. Système thérapeutique transdermique selon une ou plusieurs des revendications précédentes, **caractérisé en ce que** la couche contenant des substances actives contient un adjuvant basique libérant la substance active de la forme de sel, dans lequel au moins une substance active se trouve dans les corps creux sous la forme d'un de ses sels thérapeutiquement actifs.

11. Système thérapeutique transdermique selon une ou plusieurs des revendications précédentes, **caractérisé en ce que** la paroi des corps creux est de nature semi-perméable et libère le fluide de remplissage par une rupture de celle-ci provoquée de préférence par introduction d'eau dans les corps creux.

12. Système thérapeutique transdermique selon une ou plusieurs des revendications précédentes, **caractérisé en ce que** le diamètre extérieur des corps creux vaut de 1 nm à 1000 µm, de préférence de 10 nm à 500 µm, de préférence encore de 10 nm à 100 µm, et en particulier de préférence de 10 nm à 50 µm.

13. Système thérapeutique transdermique selon une ou plusieurs des revendications précédentes, **caractérisé en ce que** les corps creux sont de nature fragile et/ou flexible.

14. Système thérapeutique transdermique selon une ou plusieurs des revendications précédentes, **caractérisé en ce que** les corps creux sont fabriqués en fibres creuses ORMOCER.

15. Système thérapeutique transdermique selon une ou plusieurs des revendications précédentes, **caractérisé en ce que** la couche contenant des substances actives présente en outre une couche autoadhésive du côté de la peau.
